# EUROPEAN PATENT APPLICATION

(11) **EP 2 213 280 A1**
(43) Date of publication of application: **04.08.2010**
(21) Application number: 09151697.1
(22) Date of filing: 30.01.2009
(51) Int. Cl.: A61K 9/14, A61K 38/47, A61K 9/20, A61K 9/48, A61K 9/50, A61K 31/7028, A23L 1/30, A23L 1/212, A23L 1/305

(54) **Formulations comprising glucosinolate and myrosinase**

(71) Applicant: DSM IP Assets B.V., 6411 TE Heerlen (NL)
(72) Inventor: The designation of the inventor has not yet been filed
(74) Representative: Kurt, Manfred

(57) **Abstract**

The present invention relates to formulations, which comprise glucosinolate(s) such as glucoraphanin and myrosinase wherein the formulation is encapsulated and/or coated. Such a formulation can be used as such or in any kind of food or feed product.

## Description

The present invention relates to formulations, which comprise glucosinolate(s) and myrosinase. Such a formulation can be used as such or in any kind of food or feed product.

The plant family Brassicaceae (also named Cruciferae) are very well known as vegetables having positive dietary effects. Members of this plant family are for example broccoli, cabbage, cauliflower Brussels sprouts, kale and collard, romanesco, broccoflower, kohlrabi, turnip and rapeseeds.

An important species of the Brassicaceae family is Brassica oleracea. Members of that species are kale, collard, chinese broccoli, cauliflower, romanesco broccoli, broccoflower, cabbage, brussels sprouts, kohlrabi and broccoli. These plants comprise glucosinolates (especially glucoraphanin) and endogenous myrosinase enzyme (=myrosinase). Myrosinase converts glucosinolates into isothiocyanates. Such isothiocyanates (especially sulforaphane) are responsible to induce phase II enzymes, which are present in the human body and which are responsible to detoxifying carcinogens.

The glucosinolates (especially glucoraphanin) and the myrosinase enzyme are physically segregated in the plant. Therefore they cannot react with each other. The reaction starts when the plant, such as for example broccoli is chewed or cut. When the plants are cooked (which is usually the preferred way of consuming these vegetables), the myrosinase is inactivated (at least partially) and therefore the formation of sulforaphane is decreased or even stopped.

To introduce these good effects into food products it is possible to take sulforaphane as such. But this compound is not heat stable and it also has a pungent flavour.

When using glucoraphanin in food, the problem is that the conversion into sulforaphane has to be done by the bowel flora. This conversion is far less efficient than the one by myrosinase.

Because of the reactivity of the glucosinolates with the myrosinase these components are usually not formulated together in a single formulation. Two separate formulations (one with the glucosinolates and one with the myrosinase) are commonly used.

If they are formulated together, such a formulation is meant to be consumed immediately.

Therefore the goal of the present patent application was to find a composition, which comprises at least one glucosinolate compounds and myrosinase, which do not react in the composition, but which must react (shortly) after the intake.

Surprisingly, it has been found out that a formulation comprising glucosinolates and myrosinase is stable when the glucosinolate and/or the myrosinase are encapsulated or coated.

Therefore the present invention relates to a formulation comprising
(i) at least one glucosinolate and
(ii) myrosinase,
**characterized in that** component (i) and/or component (ii) are encapsulated and/or coated.

In the context of the present invention the term "stable" means that a stable formulation can be stored and used under common conditions whereby the concentrations of the two components (i) and (ii) do not decrease significantly.

The formulation according to the present invention can be any common known formulation. Preferably, a formulation according to the present invention is in a solid (or gel-like) form.

Preferably the formulation according to the present invention is in the form of a powder, a granule or a beadlet. More preferably, the formulation is a granule or a beadlet.

Especially in these cases, wherein the formulation is a used as a dietary supplement, the formulation as such can also be a galenical such as a capsule or a tablet.

Therefore, the present invention also relates to a solid formulation comprising
(i) at least one glucosinolate and
(ii) myrosinase,
**characterized in that** component (i) and/or component (ii) are encapsulated and/or coated.

Preferably the present invention relates to a granule comprising
(i) at least one glucosinolate and
(ii) myrosinase,
**characterized in that** component (i) and/or component (ii) are encapsulated and/or coated.

Preferably the present invention relates to a beadlet comprising
(i) at least one glucosinolate and
(ii) myrosinase,
**characterized in that** component (i) and/or component (ii) are encapsulated and/or coated.

Preferably the present invention relates to a galenical (capsule or table) comprising
(i) at least one glucosinolate and
(ii) myrosinase,
**characterized in that** component (i) and/or component (ii) are encapsulated and/or coated.

At least one of the essential components (i) and (ii) is encapsulated or coated. That means that either one or both of the components arte encapsulated or coated.

It is also possible that one of the components can be a mixture of encapsulated and coated particles.

It is also possible that the formulation according to the present invention is coated.

The shape of the formulation as well as the shape of the components is not essential to the invention. The same applies for the particle size of the formulation. Usually the size of the particles of a formulation is defined by the diameter of the longest dimension.

The particles of a formulation according to the present invention usually have a diameter (of the longest dimension) of 50 to 800 µm. But it is also possible that the particles are smaller or larger.

Preferred beadlets according to the present invention comprise
(i) at least one glucosinolate which is encapsulated, and
(ii) myrosinase.

Preferred beadlets according to the present invention comprise
(i) at least one glucosinolate, and
(ii) myrosinase which is encapsulated.

Preferred beadlets according to the present invention comprise
(i) at least one glucosinolate, which is coated and
(ii) myrosinase.

Preferred beadlets according to the present invention comprise
(i) at least one glucosinolate, and
(ii) myrosinase, which is coated.

Preferred beadlets according to the present invention comprise
(i) at least one glucosinolate which is encapsulated, and
(ii) myrosinase which is encapsulated.

Preferred beadlets according to the present invention comprise
(i) at least one glucosinolate which is coated, and
(ii) myrosinase, which is coated.

Preferred granules according to the present invention comprise
(i) at least one glucosinolate which is encapsulated, and
(ii) myrosinase.

Preferred granules according to the present invention comprise
(i) at least one glucosinolate, and
(ii) myrosinase which is encapsulated.

Preferred granules according to the present invention comprise
(i) at least one glucosinolate, which is coated and
(ii) myrosinase.

Preferred granules according to the present invention comprise
(i) at least one glucosinolate, and
(ii) myrosinase, which is coated.

Preferred granules according to the present invention comprise
(i) at least one glucosinolate which is encapsulated, and
(ii) myrosinase which is encapsulated.

Preferred granules according to the present invention comprise
(i) at least one glucosinolate which is coated, and
(ii) myrosinase, which is coated.

Preferred galenicals (capsules or tables) according to the present invention comprise
(i) at least one glucosinolate which is encapsulated, and
(ii) myrosinase.

Preferred galenicals (capsules or tables) according to the present invention comprise
(i) at least one glucosinolate, and
(ii) myrosinase which is encapsulated.

Preferred galenicals (capsules or tables) according to the present invention comprise
(i) at least one glucosinolate, which is coated and
(ii) myrosinase.

Preferred galenicals (capsules or tables) according to the present invention comprise
(i) at least one glucosinolate, and
(ii) myrosinase, which is coated.

Preferred galenicals (capsules or tables) according to the present invention comprise
(i) at least one glucosinolate which is encapsulated, and
(ii) myrosinase which is encapsulated.

Preferred galenicals (capsules or tables) according to the present invention comprise
(i) at least one glucosinolate which is coated, and
(ii) myrosinase, which is coated.

The glucosinolate (component (ii)) is preferably glucoraphanin.

The (molar) ratio of glucosinolate (preferably glucoraphanin) to myrosinase in the composition can vary. Usually the myrosinase is used in a very low amount (in comparison to the glucosinolates), because of its enzymatic properties. The ratio should be chosen in such a way that the glucosinolate can react completely with the enzyme in the GI tract.

Any suitable encapsulation material can be used to encapsulate component (i) and/or component (ii).

Suitable encapsulation materials must be food-grade or pharma-grade. Preferred encapsulation materials are gelatine, gum acacia, maltodextrin, proteins, Hydroxypropylmethyl- cellulose (HPMC), polyvinyl alcohols (PVA), waxes, fats, poly(methyl)acrylates (such as Eudragit^{®} from Röhm GmbH). Usually the encapsulation material is used in a large excess in comparison to the active material (glucosinolate or myrosinase). The encapsulation material can be up to 98 wt-%, based on the total weight of component (i) or component (ii) and the encapsulation material. The amount of the encapsulation material is higher when the myrosinase is encapsulated.

Any suitable coating material can be used to coat component (i) and/or component (ii). Suitable coating materials must be food-grade or pharma-grade. Preferred coating materials are gelatine, gum acacia, maltodextrin, proteins, Hydroxypropylmethyl- cellulose (HPMC), polyvinyl alcohols (PVA), waxes, fats, poly(methyl)acrylates (such as Eudragit^{®} from Röhm GmbH) and shellac. The coating material can be up to 50 wt-%, based on the total weight of component (i) or component (ii) and the encapsulation material.

Furthermore it is also possible that a formulation according to the present invention comprises auxiliaries. These auxiliaries can be useful for the formulation by improving its properties, such as physical stability, storage stability, visual perception, etc.

Auxiliaries can also be useful for the application in the food or feed product by improving the property of these compositions, physical stability, storage stability, visual perception, controlled release in the GI-tract, pH control, oxidation resistant, etc.

The concentration of these auxiliaries can vary, depending on the use of these auxiliaries.

Further embodiments of the present invention are formulations, wherein the formulation and/or the component (i) and/or component (ii) have a low water activity a_{w}.

The water activity (a_{w}) is defined as: a_{w} ≡ p/pₒ = ERH (%) /100, wherein
p is the vapour pressure of water in a material,
pₒ is the vapour pressure of pure water, and
ERH is the equilibrium relative humidity.

The water activity is a dimensionless quantity, which defines the energy status of water in a system. a_{w} is a common value to characterise the availability of water in a food product. The water activity is temperature dependent, therefore it has to be stated that all a_{w}'s listed in this patent application are measured at 25°C (degree Celsius) if not otherwise stated.

There are several methods to measure water activity: the chilled-mirror dew point technology and the capacity sensor (or resistance) technology. All a_{w} values listed in this patent application are measured using a Novasina thermoconstanter.

The formulations according to the present invention have an a_{w} of less that 0.4. Preferably less than 0.35

Preferably the a_{w} value is between 0.05 and 0.4.

The formulations according to the invention can be produced using any commonly known process.

The encapsulation process is usually done by dissolving the active compound (component (i) and/or component (ii)) together with a matrix material in water and successive removal of water e.g. by spray-drying, fluid-bed drying, spray-chilling, belt-drying, freeze-drying, vacuum-drying or other techniques.

Suitable encapsulation materials must be food-approved or pharma-grade. Preferred encapsulation materials are gelatine, gum acacia, maltodextrin, proteins, Hydroxypropylmethyl- cellulose (HPMC), polyvinyl alcohols (PVA), waxes, fats and poly(methyl)acrylates (such as Eudragit^{®} from Röhm GmbH).

The coating is usually done by fluid-bed coating. In fluid-bed coating, the core material is fluidised in an air stream and the coating material is sprayed either as melt or as solution or suspension on the core particles. With more than one coating layer, onion like structures can be obtained.

Suitable coating materials must be food-grade or pharma-grade. Preferred coating materials are gelatine, gum acacia, maltodextrin, proteins, Hydroxypropylmethylcellulose (HPMC), polyvinyl alcohols (PVA), waxes, fats, poly(methyl)acrylates (such as Eudragit^{®} from Röhm GmbH) and shellac.

The encapsulation and the coating process can be combined easily. It is possible to have an encapsulation process step and then a further encapsulation process step, or to have an encapsulation process step and then a coating process step, or to have a coating process step and then a further coating process step or to have a coating process step and then an encapsulation process step.

In the following important combined processes are disclosed:

### • Successive encapsulation and coating

Glucoraphanin is dissolved together with a suitable encapsulation material (e.g. gelatine) in water and spray-dried. These encapsulated particles are used as core material for a fluid-bed coating process. An aqueous solution of myrosinase (with a coating material) is used as spraying solution. The granule consists of a core with encapsulated glucoraphanin and a shell-layer containing myrosinase. Optionally additional separating or protecting layers may be applied. The process may also be applied vice versa with encapsulated enzyme and glucoraphanin in the coating layer.

### • Successive coating and encapsulation

Primary particles of enzyme are coated with a water insoluble coating material like fat or wax. Glucoraphanin is dissolved together with a suitable encapsulation material in water. The coated enzyme particles are dispersed into this solution without dissolving. Preferably the encapsulating solution has a high viscosity to avoid sedimentation of the enzyme particles. Water is then removed by known methods, e.g. spray-drying.

### • Multiple encapsulation

Glucoraphanin and enzyme are encapsulated and dried separately. These primary particles are dispersed in molten fat or wax. Granules are produced by spray-chilling the melt.

### • Multiple coating

In a fluid-bed processor, a suitable carrier material is fluidised. Aqueous solutions of myrosinase and of glucoraphanin (optionally with additional matrix material) are used as spraying solutions one after the other. In an onion-like structure, the carrier particle will be surrounded by shells of glucoraphanin and enzyme. Between the layers, additional separating layers may be applied. Optionally the outer layer may be a protective layer.

The formulations according to the present invention are used in food or feed products as well as they are used as dietary supplements.

Therefore a further embodiment of the present invention is the use of the formulations as described above in food or feed products.

A further embodiment is the use of the formulations as described above as dietary supplements.

Food products (for humans and/or animals) in the context of the present comprise solid food products as well as paste-like and or gel like. The food products comprise food for humans as well as for animals.

A dietary supplement, also known as food supplement or nutritional supplement, is a preparation intended to supply nutrients, such as vitamins, minerals, fatty acids or amino acids that are missing or are not consumed in sufficient quantity in a person's diet.

The food product can be in a ready-to-consume form that means a form, which is suitable to eat without further proceedings. But it is also possible that food product is a form, which needs further proceedings, like heating, dissolving, diluting, etc.

Suitable human food products can be packet soup, instant drinks, effervescent tablets, bars (cereal, chocolate), dairy products, etc.

Suitable animal food products (feed products) can be in any commonly used form.

Further embodiments are food or feed products comprising formulation as described above.

Further embodiments are dietary supplements comprising formulations as described above.

The following examples serve to illustrate the invention. The percentages are expressed in weight percentages and the temperatures are degrees Celsius, if not otherwise defined.

### Examples

### Example 1: Preparation of encapsulated Myrosinase (fat-embedding and spray-chill)

Commercially available myrosinase powder (>100U/g) is dispersed in molten cocoa butter. The molten mixture is sprayed into a spraying tower together with cold air to obtain a powder of myrosinase embedded in fat.

### Example 2: Preparation of encapsulated Myrosinase (spray-granulation and fat-coating)

Commercially available myrosinase powder (>100U/g) is dissolved in water. The aqueous solution is sprayed onto an inert carrier material (sucrose) in a fluid bed. On this primary granulate a suitable amount of molten hardening fat is sprayed in a fluid bed. The product temperature is kept below the melting point of the fat. A secondary granulate of fat-coated particles containing myrosinase is obtained.

### Example 3: Preparation of encapsulated Myrosinase (spray-granulation and enteric coating with Eudragit^{®})

Commercially available myrosinase powder (>100U/g) is dissolved in water. The aqueous solution is sprayed onto an inert carrier material (sucrose) in a fluid bed. On this primary granulate a suitable amount of Eudragit suspension is sprayed in a fluid bed. A secondary granulate containing myrosinase and coated with Eudragit is obtained.

### Example 4: Preparation of encapsulated Glucoraphanin (spray-granulation and enteric coating with Eudragit^{®})

Broccoli extract containing a suitable amount of Glucoraphanin is dissolved in water. The aqueous solution is sprayed onto an inert carrier material (sucrose) in a fluid bed. On this primary granulate a suitable amount of Eudragit suspension is sprayed in a fluid bed. A secondary granulate containing Glucoraphanin and coated with Eudragit is obtained.

### Example 5: Tablet containing Glucoraphanin and encapsulated Myrosinase

Encapsulated Myrosinase from Example 1 -Example 3 and Glucoraphanin-granulate from Example 4 are mixed with other excipients. Tablets containing 40mg Glucoraphanin and 1.5 U myrosinase are produced using a tablet press.

### Example 6: Preparation of granulate with encapsulated Glucoraphanin and Myrosinase (fat-embedding and spray-chill)

Commercially available myrosinase powder (>100U/g) and broccoli extract containing a suitable amount of Glucoraphanin are dispersed in molten cocoa butter. The molten mixture is sprayed into a spraying tower together with cold air to obtain a powder of myrosinase and Glucoraphanin embedded in fat.

### Example 7: Preparation of granulate with encapsulated Glucoraphanin and Myrosinase (successive spray granulation and coating)

An aqueous solution of myrosinase is sprayed onto an inert carrier material (sucrose) in a fluid bed. On this primary granulate, an aqueous solution of Na-carboxymethyl cellulose is sprayed as separating layer. On this secondary granulate, an aqueous solution of broccoli extract is sprayed. Finally this tertiary granulate is coated with an aqueous suspension of Eudragit.

### Example 8: Preparation of granulate with encapsulated Glucoraphanin and Myrosinase (successive spray granulation and coating)

An aqueous solution of broccoli extract is sprayed onto an inert carrier material (sucrose) in a fluid bed. On this primary granulate, an aqueous solution of Na-carboxymethyl cellulose is sprayed as separating layer. On this secondary granulate, an aqueous solution of myrosinase is sprayed. Finally this tertiary granulate is coated with an aqueous suspension of Eudragit.

## Claims

1. A formulation comprising
(i) at least one glucosinolate and
(ii) myrosinase,
**characterized in that** component (i) and/or component (ii) are encapsulated and/or coated.

2. Formulation according to claim 1, wherein the formulation is solid.

3. Formulation according to any of the preceding claims, wherein the formulation is a powder, a granule or a beadlet.

4. Formulation according to any of claims 1 to 3, wherein the formulation is a galenical, preferably a tablet.

5. Formulation according to any of the preceding claims, wherein the formulation is coated.

6. Formulation according to any of the preceding claims, comprising
(i) at least one glucosinolate which is encapsulated.

7. Formulation according to any of the claims 1 - 5, comprising
(ii) myrosinase which is encapsulated.

8. Formulation according to any of the preceding claims, comprising
(i) at least one glucosinolate which is coated.

9. Formulation according to any of the claims 1 - 5, comprising
(ii) myrosinase which is coated.

10. Formulation according to any of claims 1 - 5, comprising
(i) at least one glucosinolate which is encapsulated, and
(ii) myrosinase which is encapsulated.

11. Formulation according to any of claims 1 - 5, comprising
(i) at least one glucosinolate which is coated, and
(ii) myrosinase which is coated.

12. Formulation according to any of the preceding claims, wherein the glucosinolate is glucoraphanin.

13. Formulation according to any of the preceding claims, wherein the encapsulation and the coating materials are food-grade or pharma-grade.

14. Formulation according to any of the preceding claims, wherein the encapsulation material is chosen from the group consisting of gelatine, gum acacia, maltodextrin, proteins, Hydroxypropylmethyl- cellulose (HPMC), polyvinyl alcohols (PVA), waxes, fats and poly(methyl)acrylates.

15. Formulation according to any of the preceding claims, wherein the coating material is chosen from the group consisting of gelatine, gum acacia, maltodextrin, proteins, Hydroxypropylmethyl- cellulose (HPMC), polyvinyl alcohols (PVA), waxes, fats, poly(methyl)acrylates and shellac.

16. Formulation according to any of the preceding claims, wherein the formulation comprises auxiliaries.

17. Formulation according to any of the preceding claims, wherein component (i) and/or component (ii) have a water activity a_{w} of less than 0.4.

18. Use of a formulation according to any of claims 1 -17 in food or feed products.

19. Use of a formulation according to any of claims 1 - 17 as dietary supplement.

20. Food product, feed product or dietary supplement comprising a formulation according to claims 1-17.
